# FASCICULE DE BREVET EUROPEEN

(11) **EP 2 077 873 B1**
(45) Date de publication et mention de la délivrance du brevet: **26.09.2018**
(21) Numéro de dépôt: 07823882.1
(22) Date de dépôt: 28.09.2007
(51) Int. Cl.: A61M 5/14, A61M 5/168, G21F 5/00, G01T 1/161

(54) **UNITÉ MÉDICALE POUR LE PRÉLÈVEMENT, LE CALIBRAGE, LA DILUTION ET/OU L'INJECTION D'UN PRODUIT RADIOACTIF INJECTABLE**
MEDIZINISCHES GERÄT ZUR SAMMLUNG, KALIBRIERUNG, VERDÜNNUNG UND/ODER INJEKTION EINES INJIZIERBAREN RADIOAKTIVEN PRODUKTS
MEDICAL UNIT FOR THE COLLECTION, CALIBRATION, DILUTION AND/OR INJECTION OF AN INJECTABLE RADIOACTIVE PRODUCT

(30) Priorité: 29.09.2006 FR 0608586
(43) Date de publication de la demande: 15.07.2009
(73) Titulaire: LEMER PROTECTION ANTI-X PAR ABREVIATION SOCIETE LEMER PAX, 44470 Carquefou (FR)
(72) Inventeur: LEMER, Pierre-Marie, F-44000 Nantes (FR)
(74) Mandataire: Jacobacci Coralis Harle
(86) Numéro de dépôt international: PCT/FR2007/052048
(87) Numéro de publication internationale: WO 2008/037939

(56) Documents cités:
- DE-U1-202005 008 379
- FR-A- 2 867 294
- FR-A1- 2 739 565
- NL-A- 7 607 008
- US-A- 4 981 140
- US-A- 5 039 863
- US-A1- 2005 085 682
- US-A1- 2005 278 066
- US-A1- 2006 189 854
- US-B1- 6 468 219
- US-B2- 6 767 319

## Description

La présente invention concerne le domaine général de la médecine nucléaire. Elle concerne plus particulièrement une unité médicale employée pour le prélèvement, le calibrage, la dilution et/ou l'injection d'une substance radioactive destinée à être injectée à un patient.

Certaines substances radioactives sont particulièrement utiles dans le domaine médical, par exemple dans les procédures d'imagerie, à titre d'agents de contraste, ou comme agents thérapeutiques.

Pour limiter les doses de radiations reçues par le patient et par le personnel chargé des manipulations, on utilise des radioéléments de courtes demi-vies à usage médical, c'est-à-dire que le niveau de radiation émis par ces produits radioactifs décroît rapidement avec le temps.

Mais de tels produits radioactifs à courte demi-vie rendent problématique l'administration d'une dose appropriée au patient. Le dosage correspondant doit en effet être très précis ; il doit tenir compte du temps nécessaire pour la préparation de la dose à injecter, et aussi du temps susceptible de séparer le moment de la préparation de la dose de produit et le moment de l'injection proprement dite de cette dose au patient.

En outre, malgré le type de produits mis en oeuvre (courte demi-vie), une autre contrainte à prendre en compte concerne la radioprotection du personnel médical chargé de préparer la dose radioactive et de l'injecter au patient. Cette radioprotection doit aussi être effective pour le patient.

De manière classique, les doses à injecter sont prélevées dans une seringue munie d'un blindage approprié, placée elle-même dans une enceinte blindée équipée de moyens de mesure et de contrôle appropriés, permettant de prélever dans la seringue la dose de produit radioactif recherchée. Ensuite, un opérateur récupère la seringue blindée et il se rend auprès du patient pour réaliser l'injection.

Cependant, cette manière d'opérer n'offre pas une sécurité optimale, tant sur le plan de la radioprotection pour l'opérateur que sur le plan de la précision de la dose injectée au patient.

Le document US-6 767 319 décrit un matériel de calibrage et d'injection de produit radioactif visant à limiter l'exposition du personnel à la substance radioactive et aussi optimiser la sécurité du patient.
L'installation correspondante comprend trois enceintes radioprotectrices indépendantes, contenant respectivement :
- des moyens pour le support d'une source en produit radioactif injectable,
- des moyens pour le support d'une seringue, qui sont équipés de moyens pour la manoeuvre automatique de son piston, et qui sont associés à un dispositif de type activimètre pour la mesure en temps réel de l'activité radio-isotopique émise par le produit contenu dans la seringue, et
- un système de vannes.
Ce système de vannes est raccordé hydrauliquement, par le biais de tubulures, à l'enceinte contenant la source mère radioactive, à l'enceinte contenant la seringue, à une source de sérum physiologique et à un cathéter d'injection destiné à être connecté au patient.
Ce matériel comprend encore des moyens destinés à piloter le système de vannes et les moyens de manoeuvre du piston de seringue, cela de manière adaptée pour assurer, dans un premier temps, le prélèvement d'une dose de produit radioactif et/ou de sérum physiologique au sein de la seringue, et dans un second temps l'éjection au travers du cathéter d'injection, du produit radioactif et/ou du sérum physiologique préalablement prélevés. La dose de produit radioactif est mesurée par le dispositif activimètre au cours du prélèvement dans la seringue.

Dans ce matériel, les tubulures reliant l'enceinte contenant le système de vannes et celles contenant la seringue ou la source radioactive, ne sont pas protégées et sont source d'émissions radioactives dans l'environnement.
De plus, du fait de sa structure, le matériel correspondant est encombrant. En outre, la complexité du réseau de tubulures entraîne la présence de volumes morts importants.

La présente invention propose une unité médicale originale de calibration et d'injection de produits radioactifs, très compacte, permettant le prélèvement, la mesure et l'injection des produits avec une grande précision, en toute sécurité, et avec des volumes morts réduits.
Cette unité médicale est du type comprenant :
- des moyens pour le support d'un conteneur en matériau radioprotecteur dans lequel est logée une source ou un générateur de produit radioactif injectable,
- des moyens pour le support d'une seringue équipée d'un piston,
- un dispositif de type activimètre pour la mesure en temps réel de l'activité radio-isotopique émise par le contenu de ladite seringue, et
- un système de conduites associé à au moins une vanne pour le raccordement hydraulique de ladite source radioactive, de ladite seringue, d'une source de sérum physiologique et d'un cathéter d'injection destiné à être connecté au patient,
ladite vanne et ledit piston de seringue étant manoeuvrables pour assurer, d'une part, une aspiration dudit produit radioactif ou dudit sérum physiologique au sein de ladite seringue, et d'autre part, une éjection dudit produit radioactif, dudit sérum physiologique ou d'un mélange de ces deux produits, préalablement aspirés au sein de ladite seringue, cela au travers dudit cathéter d'injection, la dose de produit radioactif prélevée et injectée par ladite seringue étant mesurée par ledit activimètre.
Conformément à l'invention, l'unité médicale comporte encore une enceinte blindée réalisée en au moins un matériau radioprotecteur, dans laquelle sont logés le support de source radioactive, au moins une partie des moyens support de la seringue, l'activimètre, la vanne, et au moins une partie du système de conduites.
De plus, le support de seringue, la vanne et le support de source radioactive sont agencés verticalement les uns par rapport aux autres, respectivement du haut vers le bas, le support de seringue étant agencé pour porter la seringue verticalement avec son piston orienté vers le haut.
Cet agencement particulier permet à la seringue de prélèvement/injection et à la source de produit radioactif d'être très proches de la vanne, pour obtenir un ensemble très compact, avec des volumes morts minimisés.

Selon une caractéristique de réalisation, la vanne consiste en une vanne trois voies comprenant :
- une voie supérieure, destinée à être raccordée à la seringue de prélèvement et d'injection,
- une voie inférieure, destinée à être raccordée à la source de produit radioactif injectable, et
- une voie latérale, destinée à être raccordée à une première conduite connectée à la source de sérum physiologique et à une seconde conduite connectée au cathéter d'injection, lesdites conduites étant équipées chacune d'un clapet anti-retour convenablement orienté.

Dans ce cas, l'activimètre a avantageusement une forme générale tubulaire délimitant un puits central, d'axe vertical, destiné à contenir la seringue, ledit activimètre étant muni de deux ouvertures, l'une supérieure et l'autre inférieure, cette dernière étant orientée en regard de la vanne trois voies et du support de la source radioactive.

Pour réduire les volumes morts dans les conduits de ce matériel, la voie supérieure de la vanne, destinée à être raccordée à la seringue, comporte avantageusement un opercule hermétique destiné à être percé par l'aiguille équipant ladite seringue montée sur son support ; de même, la voie inférieure de la vanne, destinée à être raccordée à la source de produit radioactif est avantageusement prolongée par une aiguille destinée à percer un opercule obturant le flacon contenant ladite source radioactive.

Encore selon une caractéristique de réalisation de l'invention, les supports de source radioactive et de seringue sont portés chacun par des moyens assurant leur(s) déplacement(s) selon un axe vertical ou sensiblement vertical, cela entre deux positions :
- une première position, dans laquelle un opérateur peut charger la source radioactive et la seringue sur leurs supports respectifs, ou à l'inverse les décharger, et
- une seconde position dans laquelle la source radioactive et la seringue sont raccordées à la vanne.

Selon cette caractéristique, les moyens de déplacement du support de seringue permettent avantageusement son cheminement verticalement au travers d'un orifice ménagé dans l'enceinte blindée, entre :
- une position supérieure de chargement/déchargement, dans laquelle ledit support se situe au moins partiellement hors de ladite enceinte, et
- une position inférieure de raccordement, dans laquelle la seringue se positionne au sein du logement central de l'activimètre et est raccordée à la vanne.
De plus, le support de source radioactive chemine avantageusement au sein de l'enceinte blindée entre ses positions de chargement/déchargement et de raccordement ; cette enceinte est encore munie d'une trappe frontale pour permettre l'accès d'un opérateur au support de source radioactive au moins dans sa position de chargement/déchargement.

Encore selon une autre caractéristique, l'unité médicale comprend des moyens de commande informatiques et/ou électroniques aptes à piloter la vanne et les moyens de manoeuvre du piston de seringue, cela de manière à mettre en oeuvre les opérations de prélèvement et d'éjection par la seringue. De même, les moyens de commande informatiques/électroniques pilotent également éventuellement les moyens de déplacement du support de seringue et du support de source radioactive.

Dans ce cas, les moyens de manoeuvre du piston de la seringue sont avantageusement de type motoréducteur débrayable, contrôlés par les moyens informatiques/électroniques, pour assurer, d'une part, le prélèvement automatique d'une dose déterminée de produit radioactif au sein de la seringue et, d'autre part, pour assurer l'injection de cette dose au patient, soit automatiquement, soit manuellement. L'opérateur peut en effet, s'il le souhaite, débrayer les moyens motoréducteurs et contrôler manuellement l'injection de la dose radioactive au patient.

Selon toujours une forme de réalisation intéressante, l'enceinte se compose de trois sous-enceintes alignées verticalement les unes par rapport aux autres, à savoir :
- une sous-enceinte supérieure contenant la seringue et l'activimètre,
- une sous-enceinte intermédiaire contenant la vanne, et
- une sous-enceinte inférieure contenant la source de produit radioactif.
Ces sous-enceintes sont raccordées deux à deux par des ouvertures traversantes au travers desquelles passent certaines des conduites de raccordement hydraulique.

Pour optimiser encore le traitement des données des médicales, les moyens de commande informatiques/électroniques sont pourvus d'une connectique pour l'envoi et/ou la réception de données, en particulier pour les échanges avec un serveur informatique.

L'unité médicale selon l'invention peut être rendue mobile. Pour cela, elle est montée sur des roues avantageusement motorisées ; elle intègre éventuellement un système de géolocalisation, par exemple de type GPS.

L'invention sera encore illustrée, sans être aucunement limitée, par la description suivante d'un mode de réalisation particulier, donné uniquement à titre d'exemple et représenté sur les dessins annexés dans lesquels :
- la figure 1 est une représentation schématique, en coupe, d'une unité médicale conforme à l'invention ;
- la figure 2 est une vue en perspective de la structure externe d'une forme de réalisation possible de l'unité médicale illustrée figure 1.

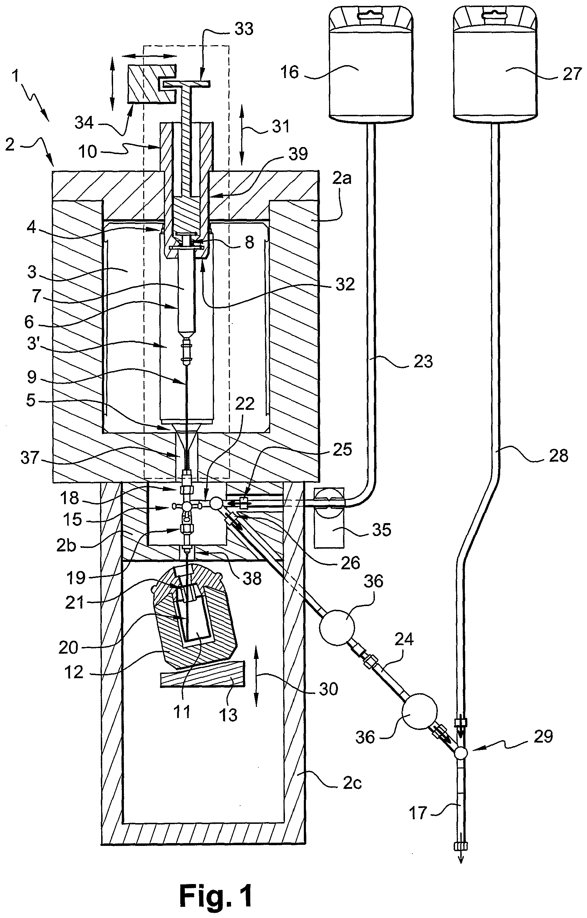

Tel que représenté sur la figure 1, l'unité médicale 1 conforme à l'invention comprend une enceinte blindée 2 réalisée en matériau radioprotecteur dans laquelle on trouve un dispositif 3 pour la mesure en temps réel de l'activité radio-isotopique (activimètre de type ACAD (marque déposée)), de forme générale cylindrique d'axe vertical, muni d'une ouverture supérieure 4 et d'une ouverture inférieure 5.

Une seringue classique 6, comprenant un corps 7, un piston 8 et une aiguille 9, est installée dans le puits de mesure 3' de l'activimètre 3 (connectée à une unité de traitement appropriée) ; cette seringue 6 est montée verticalement sur un support supérieur 10, son piston 8 étant orienté vers le haut, et donc son aiguille 9 étant orientée vers le bas.

Une source ou générateur 11 de produit radioactif est placé sous l'activimètre 3, en regard de son ouverture inférieure 5. Cette source de produit radioactif 11 est contenue dans un flacon conditionné dans un conteneur blindé 12 réalisé en matériau radioprotecteur. Le conteneur blindé 12 est logé dans l'enceinte blindée 2, posé sur un support 13.

Une vanne trois voies motorisée 15, logée dans l'enceinte blindée 2 entre la seringue 6 et le flacon de source radioactive 11, assure une connexion hydraulique appropriée entre ladite seringue 6, ledit flacon de source radioactive 11, une poche de sérum physiologique 16 (extérieure à l'enceinte blindée 2) et un cathéter 17 d'injection au patient (également extérieur à l'enceinte blindée 2). Cette vanne 15 est localisée en regard de l'ouverture inférieure 5 de l'activimètre 3, et en regard de la source radioactive 11.

La voie supérieure 18 de cette vanne trois voies 15 comporte un opercule hermétique destiné à être percé par l'aiguille 9 de la seringue 6. La voie inférieure 19 de la vanne 15 se prolonge par une aiguille 20 destinée à percer l'opercule hermétique 21 qui obture le flacon de source radioactive 11. La voie latérale 22 de la vanne 15 est connectée, par un raccordement en Y, à une tubulure 23 aboutissant à la poche de sérum physiologique 16, et à une tubulure 24 aboutissant au cathéter d'injection 17. La tubulure 23 est équipée d'un clapet anti-retour 25 empêchant un retour de liquide en direction de la poche de sérum physiologique 16. La tubulure 24 est également équipée d'un clapet anti-retour 26 imposant le passage de liquide en direction du patient.

Sur la figure 1, on remarque que le cathéter 17 est également en communication avec une seconde poche 27 de sérum physiologique, par le biais d'une tubulure 28 et d'un raccordement en Y 29.

La vanne trois voies 15 a deux positions principales : - une première mettant en communication ses voies supérieure 18 et inférieure 19 (permettant la mise en communication de la seringue 6 avec la source de produit radioactif 11 pour assurer le prélèvement d'une dose de produit radioactif dans le corps de seringue 7), et - une seconde position, mettant en communication la voie supérieure 18 et la voie latérale 22 (soit pour aspirer du sérum physiologique venant de la poche 16 dans le corps de seringue 7, lors d'une opération d'aspiration par la seringue 6, soit pour éjecter le liquide contenu dans le corps de seringue 7 dans le cathéter d'injection 17, par une manoeuvre de vidange du corps de seringue 7).
Une troisième position possible de la vanne 15 consiste à mettre en communication la source de radioéléments 11 et les tubulures 23 et 24, cela pour casser la dépression du flacon de source radioactive 11 en autorisant l'aspiration du sérum physiologique provenant de la poche 16.

La vanne trois voies 15 est montée fixe à l'intérieur de l'enceinte 2 sur l'axe vertical ou sensiblement sur l'axe vertical passant par la seringue 6 et la source 11 de produit radioactif.

Le support 13 de la source de produit radioactif 11 est mobile verticalement, conformément à la flèche d'orientation 30, sous l'action de moyens mécaniques appropriés (non représentés) actionnés manuellement (ou au pied), ou par des moyens moteurs (également non représentés) de manière à permettre l'intégration de l'aiguille 20 dans le flacon de source radioactive 11, ou le retrait de cette aiguille 20 dudit flacon.
L'opérateur manoeuvre le support mobile 13 dans cette dernière position « extraite » lorsqu'il souhaite changer la source de produit radioactif.

D'autre part, le support 10 de la seringue 6 est également mobile verticalement, conformément à la flèche d'orientation 31, sous l'action de moyens mécaniques appropriés (non représentés) actionnés manuellement ou par des moyens moteurs (également non représentés), de manière à permettre l'intégration de l'aiguille 9 de la seringue 6 dans la vanne trois voies 15, ou l'extraction de la seringue 6 au-dessus de l'activimètre 3 et hors du conteneur blindé 2, pour réaliser les opérations de mise en place et de retrait de la seringue 6.

Le support 10 de la seringue 6 est également structuré pour permettre une manoeuvre du piston 8 de la seringue depuis l'extérieur du conteneur blindé 2, alors que ladite seringue 6 est centrée dans le puits de mesure 3' de l'activimètre 3.
Pour cela, le support 10 comporte une partie cylindrique 32 en prise avec la partie arrière du corps de seringue 7, et une partie centrale 33, en forme de piston coulissant dans la partie cylindrique 32, en prise avec la partie arrière du piston de seringue 8.

Lorsque le corps de seringue 7 est en position dans le puits de mesure 3' de l'activimètre 3, l'extrémité supérieure du piston coulissant 33 est accessible depuis l'extérieur de l'enceinte blindée 2. Cette extrémité supérieure de piston 33 est associée à une motorisation débrayable 34 qui, une fois embrayée, permet l'actionnement automatique du piston de seringue 8 et qui, lorsqu'elle est débrayée, permet l'actionnement manuel de ce piston 8.
Cette particularité offre à l'opérateur un choix de gestion, automatique ou manuelle, du prélèvement de produit radioactif par la seringue 6 et/ou de l'éjection du produit dans le cathéter 17.

Sur la figure 1, on remarque encore la présence d'une électrovanne à pincement 35, positionnée sur la tubulure 23 de la poche de sérum physiologique 16. Cette électrovanne 35 a pour fonction d'empêcher la circulation intempestive de sérum physiologique au travers de la tubulure 23, avant la connexion du cathéter d'injection 17 au patient.

Sur la tubulure 24 d'alimentation du cathéter 17, on remarque aussi la présence de deux moyens anti-bulles/antibactérien 36 qui se présentent, par exemple, sous la forme de filtres, garantissant la stérilité du processus d'injection.

Toujours sur la figure 1, on remarque que l'enceinte blindée 2 se présente sous la forme de trois sous-ensembles blindés :
- un premier ensemble 2a intègre l'activimètre 3 et une partie du support de seringue 10,
- un second ensemble 2b cloisonne la vanne trois voies motorisée 15, et
- un troisième ensemble 2c cloisonne le support mobile 13 avec son conteneur blindé 12.

Les trois sous-enceintes 2a, 2b et 2c sont superposées ; la connexion entre la seringue 6 et la vanne 15 s'effectue au travers d'une ouverture 37 ménagée entre lesdits sous-ensembles 2a et 2b. La connexion entre la vanne 15 et la source de produit radioactif 11 est réalisée au travers d'une ouverture 38 ménagée entre les sous-ensembles 2b et 2c.

Le support 10 de la seringue 6 est réalisé en matériau radioprotecteur. Ses dimensions sont ajustées au mieux dans une ouverture 39 ménagée dans la partie supérieure du sous-ensemble 2a, pour obtenir une continuité de blindage en position abaissée (c'est-à-dire lorsque la seringue 6 est centrée dans le puits de mesure 3' de l'activimètre 3).

L'enceinte blindée 2 comporte encore des ouvertures appropriées pour le passage des tubulures 23 et 24 reliées, respectivement, à la poche de sérum physiologique 16 et au cathéter 17.

Les principales étapes mises en oeuvre au sein de l'unité médicale 1, pour la préparation d'une dose déterminée de produit radioactif, puis son injection au patient, sont détaillées ci-dessous.

Tout d'abord, la dose de produit radioactif à injecter au patient est préparée au sein de la seringue 6.
Pour cela, la seringue 6 (avec son piston 8 en position basse) et la source de produit radioactif 11 sont connectées à la vanne trois voies 15 ; ensuite, cette vanne 15 est pilotée de sorte que ses voies supérieure 18 et inférieure 19 soient raccordées hydrauliquement, permettant la mise en communication respectivement de l'aiguille de seringue 9 avec la source 11 de produit radioactif.
Le piston de seringue 8 est ensuite manoeuvré, vers le haut, pour aspirer la dose voulue de produit radioactif dans le corps de seringue 7, qui est mesurée en temps réel par l'activimètre 3. Cette dose est notamment fonction du poids du patient.

La dose préparée au sein de la seringue peut ensuite être administrée au patient.
A cet effet, la vanne 15 est à nouveau pilotée, cela de sorte que ses voies supérieure 18 et latérale 22 soient respectivement en communication avec l'aiguille de seringue 9, et avec les tubulures 23 et 24 (connectées à la poche 16 de sérum physiologique et au cathéter d'injection 17).
Avant la phase d'injection proprement dite, le piston de seringue 8 peut, si nécessaire, être piloté (vers le haut) pour aspirer un volume complémentaire de sérum physiologique provenant de la poche 16 ; ce volume de sérum permet de diluer le produit radioactif, et aussi d'obtenir un volume d'injection suffisant.
La seringue 6 est ensuite vidangée par le déplacement adapté du piston de seringue 8 (vers le bas). Le produit radioactif, éventuellement dilué par le volume complémentaire de sérum physiologique, chemine alors au travers de la tubulure 24 où il est filtré par les dispositifs 36, puis le long du cathéter d'injection 17 jusqu'au patient.

Suite à cette phase d'injection, l'opérateur peut éventuellement mettre en oeuvre une phase complémentaire de rinçage du corps de seringue 7, de la vanne 15, et des conduites aval 17 et 24, avec un volume adapté de sérum physiologique pour assurer l'administration au patient de la totalité de la dose radioactive souhaitée.
A cet effet, le piston de seringue 8 est manoeuvré successivement en aspiration (vers le haut) pour prélever un volume déterminé de sérum physiologique en provenance de la poche 16, puis manoeuvré en éjection (vers le bas) pour éjecter ce volume au travers de la conduite 24 et du cathéter d'éjection 17.

Lorsque l'opérateur souhaite remplacer la seringue 6 ou la source de produit radioactif 11, il lui suffit de manoeuvrer leurs structures supports respectives 10 et 13. A titre indicatif, la seringue 6 et la vanne 15 avec ses différentes conduites peuvent être remplacées suite à chaque injection. La seringue 6, d'une part, et la vanne 15 avec son aiguille 20, ses tubulures 23, 24, la poche de sérum physiologique 16 et le cathéter 17, d'autre part, constituent un ensemble stérile à usage unique, remplaçable très facilement après chaque utilisation.

Les différents cycles précités de prélèvement, de dilution et d'injection de ce matériel sont gérés par des moyens de commande électroniques/informatiques de type automate programmable, aptes à piloter automatiquement les moyens de manoeuvre 34 du piston de seringue 8 et la vanne trois voies 15, de manière appropriée. L'ensemble de ces cycles peut être totalement automatisé. En fonction des besoins, ou des souhaits de l'utilisateur, l'injection de la dose radioactive au patient peut aussi être réalisée manuellement grâce aux moyens débrayables du motoréducteur 34.

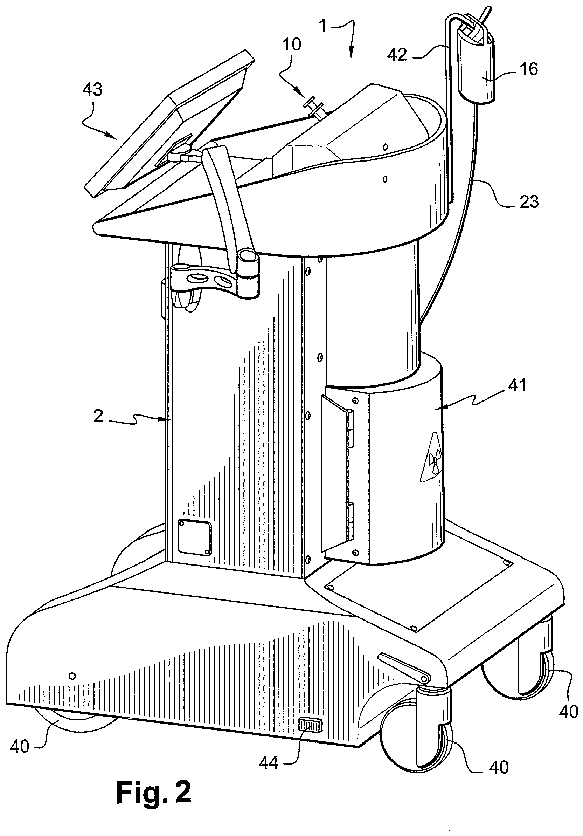

Une forme particulièrement intéressante de l'unité médicale illustrée schématiquement sur la figure 1, est représentée sur la figure 2.

Sur cette figure 2, l'enceinte blindée 2 qui intègre l'ensemble du matériel fonctionnel décrit ci-dessus, est montée sur un châssis équipé de quatre roues 40. De préférence, certaines au moins des roues 40 sont associées à une motorisation, constituant une simple assistance aux déplacements, ou assurant elle-même le déplacement autonome de l'unité mobile, pilotée à distance par un boîtier à manette adapté.

L'unité mobile 1 peut aussi intégrer un système de géolocalisation, par exemple de type GPS, pour connaître en permanence son positionnement à distance dans un bâtiment.

Dans la partie inférieure de l'enceinte 2, on remarque la présence d'une trappe blindée 41 donnant accès à l'intérieur de la sous-enceinte 2c, pour le chargement ou le déchargement sur son support 13 du conteneur blindé 12 renfermant la source de produit radioactif 11 (en particulier lorsque ce support 13 est en position basse de chargement/déchargement).

Dans la partie supérieure, on remarque le support de seringue 10, la poche de sérum physiologique 16 accrochée à un support 42, ainsi qu'un tableau 43 de commande et de visualisation, à écran tactile, intégrant l'automate programmable de gestion des cycles, ou en relation directe avec celui-ci (par exemple déporté au sein du châssis de l'unité). Ce tableau de commande, de dialogue et de visualisation 43 permet d'effectuer les opérations de calibration (mesure d'activité), et la visualisation en temps réel des diverses phases de préparation du transfert (dilution ...) et d'injection du produit radioactif.

Les moyens de commande électroniques/informatiques correspondants sont équipés d'une connectique 44 pour l'envoi et/ou la réception de données, en particulier pour réaliser certains échanges avec un serveur informatique situé à proximité ou à distance (par exemple par l'intermédiaire d'un réseau intranet ou du réseau internet), notamment pour réaliser une télémaintenance à distance et collecter certaines données concernant le patient (nécessaires notamment à la détermination de la dose de radioéléments qui doit lui être administrée).

Le châssis de l'unité 1 porte également des moyens propres d'énergie, par exemple de type batteries rechargeables, assurant l'alimentation électrique notamment des roues motorisées 40 et des moyens de commande électroniques/informatiques.

Cette unité mobile blindée 1 constitue une unité autonome permettant la calibration et l'injection de tous produits radioactifs (en particulier de FDG). Elle est très compacte du fait de la superposition de l'activimètre, de la vanne trois voies et de la source de produit radioactif sur le même axe vertical ou sensiblement sur le même axe vertical, et du fait de la superposition des sous-enceintes 2a, 2b et 2c. Cette unité permet un prélèvement, une mesure et une injection en toute sécurité.

## Revendications

1. Unité médicale pour le prélèvement, le calibrage, la dilution et/ou l'injection d'un produit radioactif, injectable à un patient, laquelle unité (1) comprend au moins :
- des moyens (13) pour le support d'un conteneur (12) en matériau radioprotecteur dans lequel est logée une source ou un générateur de produit radioactif injectable (11),
- des moyens (10) pour le support d'une seringue (6) équipée d'un piston (8),
- un dispositif (3) de type activimètre pour la mesure en temps réel de l'activité radio-isotopique émise par le contenu de ladite seringue (6), et
- un système de conduites (9, 20, 23, 24) associé à au moins une vanne (15) pour le raccordement hydraulique de ladite source radioactive (11), de ladite seringue (6), d'une source de sérum physiologique (16) et d'un cathéter d'injection (17) destiné à être connecté au patient,
ladite vanne (15) et ledit piston de seringue (8) étant manoeuvrables pour assurer, d'une part, une aspiration dudit produit radioactif (11) ou dudit sérum physiologique (16) au sein de ladite seringue (6), et d'autre part, une éjection dudit produit radioactif (11), dudit sérum physiologique ou d'un mélange de ces deux produits, préalablement aspiré(s) au sein de ladite seringue (6), cela au travers dudit cathéter d'injection (17), la dose de produit radioactif prélevée et injectée par ladite seringue (6) étant mesurée par ledit activimètre (3),
**caractérisée en ce qu'**elle comporte une enceinte blindée (2) réalisée en au moins un matériau radioprotecteur, dans laquelle sont logés ledit support (13) de source radioactive (11), au moins une partie des moyens supports (10) de la seringue (6), ledit activimètre (3), ladite vanne (15) et au moins une partie dudit système de conduites (9, 20, 23, 24), et **en ce que** ledit support de seringue (10), ladite vanne (15) et ledit support (13) de source radioactive (11) sont agencés verticalement les uns par rapport aux autres, respectivement du haut vers le bas, ledit support de seringue (10) étant agencé pour porter ladite seringue (6) avec son piston (8) orienté vers le haut.

2. Unité médicale selon la revendication 1, **caractérisée en ce que** la vanne (15) consiste en une vanne trois voies comprenant :
- une voie supérieure (18), destinée à être raccordée à la seringue (6) de prélèvement et d'injection,
- une voie inférieure (19), destinée à être raccordée à la source de produit radioactif injectable (11), et
- une voie latérale (22), destinée à être raccordée à une première conduite (23) connectée à la source de sérum physiologique (16) et à une seconde conduite (24) connectée au cathéter d'injection (17), lesdites conduites (23, 24) étant équipées chacune d'un clapet anti-retour (25, 26) convenablement orienté.

3. Unité médicale selon la revendication 2, **caractérisée en ce que** l'activimètre (3) a une forme générale tubulaire délimitant un puits central (3') d'axe vertical, destiné à contenir la seringue (6), ledit activimètre (3) étant muni de deux ouvertures, l'une supérieure (4) et l'autre inférieure (5), cette dernière étant orientée en regard de la vanne trois voies (15) et du support (13) de la source radioactive (11).

4. Unité médicale selon l'une quelconque des revendications 2 ou 3, **caractérisée en ce que** la voie supérieure (18) de la vanne (15), destinée à être raccordée à la seringue (6), comporte un opercule hermétique destiné à être percé par l'aiguille (9) équipant ladite seringue (6).

5. Unité médicale selon l'une quelconque des revendications 2 à 4, **caractérisée en ce que** la voie inférieure (19) de la vanne (15), destinée à être raccordée à la source de produit radioactif injectable (11), est prolongée par une aiguille (20) destinée à percer un opercule (21) obturant le flacon contenant ladite source radioactive (11).

6. Unité médicale selon l'une quelconque des revendications 1 à 5, **caractérisée en ce que** les supports (13, 10) de source radioactive (11) et de seringue (6) sont portés chacun par des moyens assurant leur(s) déplacement(s) selon un axe vertical ou sensiblement vertical, cela entre deux positions :
- une première position, dans laquelle un opérateur peut charger la source radioactive (11) et la seringue (6) sur leurs supports respectifs (13, 10), ou à l'inverse les décharger, et
- une seconde position dans laquelle la source radioactive (11) et la seringue (6) sont raccordées à la vanne (15).

7. Unité médicale selon la revendication 6, **caractérisée en ce que** les moyens de déplacement du support de seringue (10) permettent son cheminement verticalement au travers d'un orifice (39) ménagé dans l'enceinte blindée (2), entre :
- une position supérieure de chargement/déchargement, dans laquelle ledit support (10) se situe au moins partiellement hors de ladite enceinte (2), et
- une position inférieure de raccordement, dans laquelle la seringue (6) se positionne au sein du puits central (3') de l'activimètre (3) et est raccordée à la vanne (15).

8. Unité médicale selon l'une quelconque des revendications 6 ou 7, **caractérisée en ce que** le support (13) de source radioactive (11) chemine au sein de l'enceinte blindée (2) entre ses positions de chargement/déchargement et de raccordement, ladite enceinte (2) étant encore munie d'une trappe (41) frontale pour permettre l'accès d'un opérateur audit support (13) de source radioactive (11) au moins dans sa position de chargement/déchargement.

9. Unité médicale selon l'une quelconque des revendications 1 à 8, **caractérisée en ce qu'**elle comprend encore des moyens de commande informatiques et/ou électroniques aptes à piloter la vanne (15) et les moyens (33, 34) de manoeuvre du piston de seringue (8), cela de manière à mettre en oeuvre les opérations de prélèvement et d'éjection par ladite seringue (6), lesquels moyens de commande informatiques/électroniques pilotent également éventuellement les moyens de déplacement du support de seringue (10) et du support de source radioactive (13).

10. Unité médicale selon la revendication 9, **caractérisée en ce que** les moyens de manoeuvre du piston (8) de la seringue (6) sont de type motoréducteurs débrayables (34), contrôlés par les moyens de commande informatiques/électroniques, pour assurer, d'une part, le prélèvement automatique d'une dose déterminée de produit radioactif au sein de ladite seringue (6), et d'autre part, pour assurer l'injection de cette dose au patient, soit automatiquement, soit manuellement.

11. Unité médicale selon l'une quelconque des revendications 1 à 10, **caractérisée en ce que** l'enceinte (2) se compose de trois sous-enceintes (2a, 2b, 2ç) alignées verticalement les unes par rapport aux autres, à savoir - une sous-enceinte supérieure (2a) contenant la seringue (6) et l'activimètre (3), - une sous-enceinte intermédiaire (2b) contenant la vanne (15), et - une sous-enceinte inférieure (2c) contenant la source de produit radioactif (11), lesquelles sous-enceintes (2a, 2b, 2c) sont raccordées deux à deux par des ouvertures traversantes (37, 38) au travers desquelles passent certaines des conduites (9, 20) de raccordement hydraulique.

12. Unité médicale selon l'une quelconque des revendications 1 à 11, **caractérisée en ce que** les moyens de commande informatiques/électroniques sont pourvus d'une connectique (44) pour l'envoi et/ou la réception de données, en particulier pour les échanges avec un serveur informatique.

13. Unité médicale selon l'une quelconque des revendications 1 à 12, **caractérisée en ce qu'**elle est montée sur des roues (40) pour la rendre mobile, et **en ce qu'**elle intègre éventuellement un système de géolocalisation, par exemple de type GPS.

## Patentansprüche

1. Medizinisches Gerät zur Sammlung, Kalibrierung, Verdünnung und/oder Injektion eines einem Patienten injizierbaren radioaktiven Produkts, wobei das Gerät (1) wenigstens
- Mittel (13) zum Halten eines Behälters (12) aus Strahlenschutzmaterial, in dem eine Quelle oder ein Erzeuger eines injizierbaren radioaktiven Produkts (11) untergebracht ist,
- Mittel (10) zum Halten einer mit einem Kolben (8) ausgestatteten Spritze (6),
- eine Vorrichtung (3) vom Typ eines Strahlungsmessers zum Messen in Echtzeit der vom Inhalt der Spritze (6) abgegebenen radioisotopen Strahlung und
- ein wenigstens einem Ventil (15) zugeordnetes System von Leitungen (9, 20, 23, 24) zum flüssigkeitsmäßigen Anschließen der radioaktiven Quelle (11), der Spritze (6), einer Quelle physiologischen Serums (16) und eines mit dem Patienten zu verbindenden Injektionskatheters (17)
aufweist,
wobei das Ventil (15) und der Kolben (8) der Spritze betätigbar sind, um einerseits ein Einsaugen des radioaktiven Produkts (11) oder des physiologischen Serums (16) in die Spritze (6) und andererseits ein Ausstoßen des radioaktiven Produkts (11), des physiologischen Serums oder einer Mischung dieser beiden Produkte, das bzw. die zuvor in die Spritze (6) eingesaugt worden ist bzw. sind, und zwar durch den Injektionskatheter (17), sicherzustellen, wobei die Dosis des entnommenen und durch die Spritze (6) injizierten radioaktiven Produkts durch die Strahlenmeßvorrichtung (3) gemessen wird, **dadurch gekennzeichnet, daß** es eine aus wenigstens einem Strahlenschutzmaterial gefertigte abgeschirmte Hülle (2) aufweist, in der die Halterung (13) der radioaktiven Quelle (11), wenigstens ein Teil der Haltemittel (10) für die Spritze (6), der Strahlenmesser (3), das Ventil (15) und wenigstens ein Teil des Systems von Leitungen (9, 20, 23, 24) untergebracht sind, und daß die Haltemittel (10) für die Spritze, das Ventil (15) und die Halterung (13) der radioaktiven Quelle (11) senkrecht übereinander, und zwar von oben nach unten, angeordnet sind, wobei die Haltemittel (10) für die Spritze dazu ausgelegt sind, die Spritze (6) mit dem Kolben (8) nach oben gerichtet zu tragen.

2. Medizinisches Gerät gemäß Anspruch 1, **dadurch gekennzeichnet, daß** das Ventil (15) aus einem Drei-Wege-Ventil besteht, das
- einen oberen Weg (18), der dazu bestimmt ist, mit der Entnahme- und Injektionsspritze (6) verbunden zu werden,
- einen unteren Weg (19), der dazu bestimmt ist, mit der Quelle des radioaktiven injizierbaren Produkts verbunden zu werden, und
- einen seitlichen Weg (22), der dazu bestimmt ist, an eine erste, mit der Quelle physiologischen Serums (16) verbundene Leitung (23) und an eine zweite, mit dem Injektionskatheter (17) verbundene Leitung (24) angeschlossen zu werden,
aufweist, wobei jede der Leitungen (23, 24) mit einem geeignet orientierten Rückschlagventil (25, 26) ausgestattet ist.

3. Medizinisches Gerät gemäß Anspruch 2, **dadurch gekennzeichnet, daß** der Strahlenmesser (3) eine im Wesentlichen rohrartige Form aufweist, die einen zentralen Hohlraum (3') mit senkrechter Achse umgrenzt, der dazu bestimmt ist, die Spritze (6) aufzunehmen, wobei der Strahlenmesser (3) mit zwei Öffnungen versehen ist, einer oberen (5) und einer unteren (6), wobei letztere zum Drei-Wege-Ventil (15) und zur Halterung (13) der radioaktiven Quelle (11) gerichtet ist.

4. Medizinisches Gerät gemäß einem der Ansprüche 2 oder 3, **dadurch gekennzeichnet, daß** der obere Weg (18) des Ventils (15), der dazu bestimmt ist, mit der Spritze (6) verbunden zu werden, einen hermetischen Deckel aufweist, der dazu bestimmt ist, von der Nadel (9) durchstochen zu werden, mit der die Spritze (6) ausgestattet ist.

5. Medizinisches Gerät gemäß einem der Ansprüche 2 bis 4, **dadurch gekennzeichnet, daß** der untere Weg (19) des Ventils (15), der dazu bestimmt ist, mit der Quelle radioaktiven injizierbaren Produkts verbunden zu werden, mit einer Nadel (20) verlängert ist, die dazu bestimmt ist, einen Deckel (21) zu durchstechen, der die die radioaktive Quelle (11) enthaltende Flasche verschließt.

6. Medizinisches Gerät gemäß einem der Ansprüche 1 bis 5, **dadurch gekennzeichnet, daß** die Mittel (13, 10) zum Halten der radioaktiven Quelle (11) und der Spritze (6) jeweils durch Mittel gehalten sind, die deren Verlagerung(en) entlang einer senkrechten oder im Wesentlichen senkrechten Achse sicherstellen, und zwar zwischen zwei Stellungen:
- einer ersten Stellung, in der ein Bediener die radioaktive Quelle (11) und die Spritze (6) auf deren jeweiliges Haltemittel (13, 10) setzen bzw. umgekehrt von diesem abnehmen kann, und
- einer zweiten Stellung, in der die radioaktive Quelle (11) und die Spritze (6) mit dem Ventil (15) verbunden sind.

7. Medizinisches Gerät gemäß Anspruch 6, **dadurch gekennzeichnet, daß** die Mittel zum Verlagern der Haltemittel (10) für die Spritze einen senkrechten Weg durch ein in der abgeschirmten Hülle (2) eingerichtetes Loch (39) ermöglichen, und zwar zwischen
- einer oberen Stellung zum Aufsetzen bzw. Abnehmen, in der sich das Haltemittel (10) wenigstens teilweise außerhalb der Hülle (2) befindet, und
- einer unteren Stellung der Verbindung, in der sich die Spritze (6) im zentralen Hohlraum (3') des Strahlenmessers (3) befindet und mit dem Ventil (15) verbunden ist.

8. Medizinisches Gerät gemäß einem der Ansprüche 6 oder 7, **dadurch gekennzeichnet, daß** sich die Haltemittel (13) für die radioaktive Quelle (11) zwischen deren Stellungen zum Aufsetzen bzw. Abnehmen und zum Verbinden in der abgeschirmten Hülle (2) bewegen, wobei die Hülle (2) mit einer Frontklappe (41) versehen ist, um den Zugriff eines Bedieners zum Haltemittel (13) für die radioaktive Quelle (11) wenigstens in deren Stellung zum Aufsetzen bzw. Abnehmen zu ermöglichen.

9. Medizinisches Gerät gemäß einem der Ansprüche 1 bis 8, **dadurch gekennzeichnet, daß** es außerdem Informatik- und/oder elektronische Steuermittel aufweist, die dazu ausgelegt sind, das Ventil (15) und die Mittel (33, 34) zum Betätigen des Kolbens (8) der Spritze zu steuern, und dies um die Vorgänge des Entnehmens und des Ausstoßens durch die Spritze (6) auszuführen, wobei die Informatik- und/oder elektronischen Steuermittel außerdem eventuell die Mittel zum Verlagern der Haltemittel (10) für die Spritze und der Haltemittel (13) für die radioaktive Quelle steuern.

10. Medizinisches Gerät gemäß Anspruch 9, **dadurch gekennzeichnet, daß** die Mittel zum Betätigen des Kolbens (8) der Spritze (6) vom Typ abkoppelbarer Getriebemotoren (34) sind, die von den Informatik- und/oder elektronischen Steuermitteln gesteuert werden, um einerseits die automatische Entnahme einer bestimmten Menge radioaktiven Produkts in die Spritze (6) sicherzustellen und um andererseits sicherzustellen, daß diese Menge dem Patienten automatisch oder von Hand injiziert wird.

11. Medizinisches Gerät gemäß einem der Ansprüche 1 bis 10, **dadurch gekennzeichnet, daß** sich die Hülle (2) aus drei senkrecht übereinander ausgerichteten Unterhüllen (2a, 2b, 2c) zusammensetzt, und zwar
- einer oberen Unterhülle (2a), die die Spritze (6) und den Strahlenmesser (3) enthält,
- einer mittleren Unterhülle (2b), die das Ventil (15) enthält, und
- einer unteren Unterhülle (2c), die die Quelle radioaktiven Produkts enthält,
wobei die Unterhüllen (2a, 2b, 2c) paarweise mit Durchgangsöffnungen (37, 38) verbunden sind, durch die gewisse der Leitungen (9, 20) zum flüssigkeitsmäßigen Verbinden führen.

12. Medizinisches Gerät gemäß einem der Ansprüche 1 bis 11, **dadurch gekennzeichnet, daß** die Informatik- und/oder elektronischen Steuermittel mit Anschlußmitteln (44) zum Absenden und/oder Empfangen von Daten versehen sind, insbesondere für den Austausch mit einem Informatikserver.

13. Medizinisches Gerät gemäß einem der Ansprüche 1 bis 12, **dadurch gekennzeichnet, daß** es auf Rädern (40) montiert ist, um es beweglich zu machen, und daß es außerdem eventuell ein Geolokalisierungssystem, zum Beispiel vom GPS-Typ, aufweist.

## Claims

1. Medical unit for withdrawal, calibration, dilution and/or injection of a radioactive product that can be injected to a patient, which unit (1) comprises at least:
- means (13) for supporting a container (12) made of a radioprotective material in which is accommodated a source or a generator of injectable radioactive product (11),
- means (10) for supporting a syringe (6) equipped with a plunger (8),
- a device (3) of the activimeter type for measuring in real time the radioisotopic activity from the contents of said syringe (6), and
- a system of pipes (9, 20, 23, 24) associated with at least one valve (15) for hydraulic connection of said radioactive source (11), of said syringe (6), of a source of physiological saline solution (16) and of an injection catheter (17) intended for being connected to the patient,
wherein said valve (15) and said syringe plunger (8) can be operated to ensure, on the one hand, aspiration of said radioactive product (11) or said physiological saline solution (16) into said syringe (6), and on the other hand, ejection, through said injection catheter (17), of said radioactive product (11), said physiological saline solution or a mixture of both, beforehand aspirated into said syringe (6), the dose of radioactive product withdrawn and injected by said syringe (6) being measured by said activimeter (3),
**characterized in that** it comprises a shielded enclosure (2) made of at least one radioprotective material, in which are accommodated said support (13) of the radioactive source (11), at least one part of the means (10) for supporting the syringe (6), said activimeter (3), said valve (15) and at least one part of said system of pipes (9, 20, 23, 24), and **in that** the syringe support (10), said valve (15) and said support (13) of the radioactive source (11) are arranged vertically relative to one another, respectively from top to bottom, said syringe support (10) being arranged to carry said syringe (6) with the plunger (8) thereof oriented upward.

2. Medical unit according to claim 1, **characterized in that** the valve (15) consists in a three-way valve comprising :
- an upper way (18), intended for being connected to the withdrawal and injection syringe (6),
- a lower way (19), intended for being connected to the source of injectable radioactive product (11), and
- a side way (22), intended for being connected to a first pipe (23) connected to the source of physiological saline solution (16) and to a second pipe (24) connected to the injection catheter (17), said pipes (23, 24) being each equipped with a suitably oriented check valve (25, 26).

3. Medical unit according to claim 2, **characterized in that** the activimeter (3) has a generally tubular shape circumscribing a vertical-axis central well (3') intended for containing the syringe (6), said activimeter (3) being provided with two openings, an upper one (4) and a lower one (5), the latter being directed opposite the three-way valve (15) and the support (13) of the radioactive source (11).

4. Medical unit according to any one of claims 2 or 3, **characterized in that** the upper way (18) of the valve (15), intended for being connected to the syringe (6), comprises a tight membrane seal intended for being pierced by the needle (9) fitting said syringe (6).

5. Medical unit according to any one of claims 2 to 4, **characterized in that** the lower way (19) of the valve (15), intended for being connected to the source of injectable radioactive product (11) is extended with a needle (20) intended for piercing a membrane seal (21) closing the vial that contains said radioactive source (11).

6. Medical unit according to any one of claims 1 to 5, **characterized in that** the supports (13, 10) for the radioactive source (11) and the syringe (6) are each carried by means ensuring displacement(s) thereof along a vertical or substantially vertical axis, between two positions:
- a first position, in which an operator can install the radioactive source (11) and the syringe (6) onto the respective supports (13, 10) thereof, or inversely to remove them, and
- a second position, in which the radioactive source (11) and the syringe (6) are connected to the valve (15).

7. Medical unit according to claim 6, **characterized in that** the means for displacing the syringe support (10) enable the later to travel vertically through an orifice (39) arranged in the shielded closure (2), between :
- an upper installing/removing position, in which said support (10) is located at least partially outside said enclosure (2), and
- a lower connecting position, in which the syringe (6) is positioned inside the central well (3') of the activimeter (3) and is connected to the valve (15).

8. Medical unit according to any one of claims 6 or 7, **characterized in that** the support (13) of the radioactive source (11) travels inside the shielded enclosure (2) between the installing/removing and connecting positions thereof, said enclosure (2) being further provided with a front trap door (41) for enabling an operator to reach said support (13) of the radioactive source (11) at least when the latter is in the installing/removing position thereof.

9. Medical unit according to any one of claims 1 to 8, **characterized in that** it further comprises computer and/or electronic control means able to drive the valve (15) and the means (33, 34) for operating the syringe plunger (8), in order for the withdrawal and ejection operations of the syringe (6) to be performed, which computer/electronic control means possibly also drive the means for displacing the syringe support (10) and the radioactive source support (13).

10. Medical unit according to claim 9, **characterized in that** the means for operating the plunger (8) of the syringe (6) are of the disengageable gear motor (34) type, controlled by the computer/electronic control means, to ensure, on the one hand, automatic withdrawal of a definite dose of radioactive product into said syringe (6), and on the other hand, injection of this dose to the patient, either automatically or manually.

11. Medical unit according to any one of claims 1 to 10, **characterized in that** the enclosure (2) consists in three sub-enclosures (2a, 2b, 2c) aligned vertically relative to one another, namely: - an upper sub-enclosure (2a) containing the syringe (6) and the activimeter (3), - an intermediate sub-enclosure (2b) containing the valve (15), and a lower sub-enclosure (2c) containing the source of radioactive product (11), which sub-enclosures (2a, 2b, 2c) are connected together two by two via through-openings (37, 38) through which pass some of the hydraulic connecting pipes (9, 20).

12. Medical unit according to any one of claim 1 to 11, **characterized in that** the computer/electronic control means are provided with connectics (44) for sending and/or receiving data, in particular for exchanges with a computer server.

13. Medical unit according to any one of claim 1 to 12, **characterized in that** it is mounted on wheels (40) to be rendered mobile, and **in that** it possibly integrates a geolocation system, for example of the GPS type.
